# EUROPÄISCHE PATENTSCHRIFT

(11) **EP 3 996 637 B1**
(45) Veröffentlichungstag und Bekanntmachung des Hinweises auf die Patenterteilung: **28.02.2024**
(21) Anmeldenummer: 20734475.5
(22) Anmeldetag: 15.06.2020
(51) Int. Cl.: A61F 5/01

(54) **VERSCHLUSS FÜR EINE ORTHESE**
CLOSURE FOR AN ORTHOSIS
ÉLÉMENT DE FERMETURE POUR UNE ORTHÈSE

(30) Priorität: 08.07.2019 DE 202019103735 U
(43) Veröffentlichungstag der Anmeldung: 18.05.2022
(73) Patentinhaber: Thuasne Deutschland GmbH, 30938 Burgwedel (DE)
(72) Erfinder: SCHÖBEL, Herbert, 30938 Burgwedel (DE)
(74) Vertreter: Jabbusch, Matthias
(86) Internationale Anmeldenummer: PCT/EP2020/066457
(87) Internationale Veröffentlichungsnummer: WO 2021/004726

(56) Entgegenhaltungen:
- WO-A1-2013/156351
- KR-A- 20180 128 154
- US-A1- 2009 099 495
- US-A1- 2014 123 440
- US-B1- 7 785 283

## Beschreibung

Die Erfindung betrifft einen Verschluss für eine Orthese, insbesondere einen Schnellverschluss für eine Orthese, aufweisend ein Aufsteckteil und einen Halteabschnitt, wobei das Aufsteckteil mit dem Halteabschnitt lösbar befestigbar ist und mit den weiteren Merkmalen des Patentanspruchs 1.

Verschlüsse für Orthesen werden vor allem für solche Orthesen verwendet, die ein häufiges Anlegen und Ablegen dieser erfordern, beispielsweise wenn die Orthese nur während Belastung eines jeweiligen Körperteils getragen werden soll. Der Halteabschnitt ist dabei zumeist ein an oder auf einem Grundkörper der Orthese angeordnetes Aufnahmeteil, das clip-artig mit dem Aufsteckteil zusammenwirkt, wobei das Aufsteckteil in oder an dem als Halteabschnitt ausgebildeten Aufnahmeteil verrastbar ist. Das Aufsteckteil ist weiterhin häufig einem beweglichen und/oder verstellbaren Teil der Orthese zugeordnet, mit dem die Orthese an dem jeweiligen Körperteil festlegbar ist. Bei bekannten Orthesen sind Aufsteckteil und Aufnahmeteil des Verschlusses dabei jeweils zugerüstete Teile, welche an oder auf der eigentlichen Orthese bzw. dem Grundkörper der Orthese befestigt werden. Eine Befestigung des Verschlusses kann dann unter anderem durch Niete, textile Schweiß- oder Nahtverbindungen erfolgen. Da die bekannten Verschlüsse jedoch zumeist oberflächlich auf dem Grundkörper der Orthese angeordnet sind, können diese leichter herausreißen oder sich anderweitig lösen. Eine gattungsgemäße Orthese ist aus der US 2009/099495 A1 bekannt. Weitere gattungsgemäße Orthesen werden in den Druckschriften KR 2018 0128154 A und US 2014/123440A1 beschrieben. US 2014/123440A1 wird als nächstliegender Stand der Technik angesehen und offenbart die Merkmale des Oberbegriffs des Anspruchs 1.

Ein Schnellverschluss ist zudem aus der WO 2013/156351 A1 bekannt. Ein weiterer Orthesenverschluss ist in der US 7785283 B1 beschrieben.

Aufgabe der Erfindung ausgehend von diesem Stand der Technik ist es daher, eine kompaktere Konstruktion zu realisieren.

Die Lösung dieser Aufgabe erfolgt mit einem Verschluss mit den Merkmalen des Anspruchs 1 sowie einer Orthese mit den Merkmalen des Anspruchs 12. Der Verschluss für eine Orthese, insbesondere der Schnellverschluss für eine Orthese, aufweisend ein Aufsteckteil und einen Halteabschnitt, wobei das Aufsteckteil mit dem Halteabschnitt lösbar befestigbar ist, zeichnet sich dadurch aus, dass der Aufsteckteil wenigstens zwei Stecklaschen aufweist, die an einem ersten Ende an einer Basis des Aufsteckteils miteinander verbunden sind und einen Aufnahmebereich des Aufsteckteils zu zwei einander gegenüberliegenden Seiten begrenzen, dass der Halteabschnitt derart ausgebildet ist, dass dieser bei an diesem befestigtem Aufsteckteil in dem Aufnahmebereich zwischen den zwei Stecklaschen gehalten ist, insbesondere in dem Aufnahmebereich zwischen den zwei Stecklaschen verklemmt ist, dass der Halteabschnitt Teil eines Rahmens oder eines Gerüsts oder einer Schiene oder eines anderen Teils einer Orthese ist, dass der Halteabschnitt wenigstens eine Aussparung aufweist, dass wenigstens eine der Stecklaschen wenigstens ein Rastelement aufweist, und dass das Rastelement bei auf den Halteabschnitt aufgeschobenem Aufsteckteil in die einen Hinterschnitt ausbildende Aussparung eingreift.

Indem das Aufsteckteil über die Stecklaschen und den Halteabschnitt direkt mit dem Rahmen, dem Gerüst oder der Schiene oder einem anderen Teil der Orthese verbindbar ist, ist auf einfache Weise erreicht, dass der Verschluss bestmöglichst in die Orthese integriert ist. Das Aufsteckteil greift dabei unmittelbar an "tragende Teile" der Orthese, wie den Rahmen, das Gerüst oder die Schiene oder einem anderen Teil, insbesondere formstabilen Teil, an. Dadurch ist ein besonders sicherer Halt des Verschlusses an der Orthese und der Orthese an einem jeweiligen Körperteil erreicht. Durch Verwendung des Rahmens, des Gerüsts oder der Schiene der Orthese als Halteabschnitt ist zudem eine Reduzierung der für den Verschluss benötigten Teile erreicht, da ein gesondertes Aufnahmeteil des Verschlusses für das Aufsteckteil entfällt. Die beiden Stecklaschen ermöglichen weiter eine einfache Führung des Aufsteckteils, so dass der Verschluss auch von ungeübten Personen gehandhabt werden kann.

Das Rastelement kann auf einfache Weise eine in den Aufnahmebereich hineinragende Rastnase sein. In weiterer Ausgestaltung weist das Rastelement, insbesondere die Rastnase, wenigstens einen in Richtung der Basis des Aufsteckteils weisenden Vorsprung auf, wobei zwischen einander zugewandten Flächen der Stecklasche an der das Rastelement angeordnet ist und des Rastelements selbst ein Winkel kleiner 90° ausgebildet ist. Durch den in Richtung der Basis des Aufsteckteils weisenden Vorsprung ist auf einfache Weise einer stärkeren Abnutzung und Beanspruchung des Rastelements entgegengewirkt. Auch wenn sich der Vorsprung durch Öffnen und Schließen des Verschlusses zunehmend abnutzt und damit der Winkel zwischen den einander zugewandten Flächen der Stecklasche und des Rastelements vergrößert, kann so über eine gewöhnliche Nutzungszeit ein sicheres Verrasten des Verschlusses gewährleistet werden.

Um eine verbesserte Führung des Aufsteckteils beim Schließen des Verschlusses zu erreichen, kann weiterhin vorgesehen sein, dass das Rastelement mit seiner der gegenüberliegend angeordneten Stecklasche zugewandten Rückseite in Richtung Basis zunehmend breiter wird und einen Keil ausbildet. Die Rückseite des Rastelements weist dabei zu der Stecklasche, an der das Rastelement angeordnet ist, einen flacheren Winkel auf als die vorgenannte, der Stecklasche zugewandte Fläche zum Vorsprung hin, um ein Aufschieben des Aufsteckteils mit dem Rastelement auf den Halteabschnitt zu vereinfachen.

Um ein einfaches Öffnen des Verschlusses zu gewährleisten und so die Handhabbarkeit zu verbessern, ist weiter vorgesehen, dass wenigstens eine der Stecklaschen an ihrem, dem ersten Ende entgegen gesetzten freien Ende als Anfasser ausgebildet ist. Der Anfasser ist in weiterer Ausgestaltung ein nach außen, d.h. vom Aufnahmebereich des Aufsteckteils weg gebogener, Abschnitt der Stecklasche. Dieser gebogene Abschnitt ermöglicht ein vereinfachtes Ergreifen der Stecklasche beim Aufbiegen der Stecklasche nach außen. Beim Aufbiegen der Stecklasche wird eine Vergrößerung des Abstands der Stecklaschen zueinander und damit eine Vergrößerung des Aufnahmebereichs zwischen den Stecklaschen erreicht, so dass das Rastelement aus der Aussparung gleiten kann. Gemäß einer vorteilhaften Ausgestaltung ist der Anfasser daher an der Stecklasche angeordnet, die auch das Rastelement aufweist.

Weiter ist nach einer Weiterbildung wenigstens eine der Stecklaschen länger als die andere Stecklasche. Ein solcher längerer, überstehender Stecklaschen-Abschnitt kann als Orientierungshilfe bzw. Einführungshilfe beim Befestigen des Aufsteckteils dienen. Ein Anwender legt diesen verlängerten Stecklaschen-Abschnitt auf den Halteabschnitt und hat in der Höhe das Aufsteckteil direkt positioniert.

Insbesondere kann die Stecklasche mit dem Anfasser auch länger ausgebildet sein als die Stecklasche ohne Anfasser, um einer den Verschluss bzw. die Orthese benutzenden Person eine haptische und optische Orientierungshilfe während des Gebrauchs zu geben.

Das Aufsteckteil ist nach einer Weiterbildung dem Teil einer Orthese zugeordnet, der dem Festlegen der Orthese an einem Körperteil dient. Ein solcher Teil einer Orthese ist beispielsweise eine Schlaufe, ein Riemen, ein Klettband oder dergleichen, die jeweils einstellbar sind und eine genaue Anpassung der Orthese an einem bestimmten Körperteil eines Patienten ermöglichen. Wobei die Aufzählung nicht abschließend ist.

Um das Aufsteckteil jetzt mit dem vorgenannten Teil oder den vorgenannten Teilen zum Festlegen der Orthese, wie beispielsweise einer Schlaufe, einem Riemen, einem Klettband oder dergleichen, zu verbinden, kann das Aufsteckteil wenigstens eine Schlaufenaufnahme aufweisen. Eine solche Kombination aus Aufsteckteil und Schlaufe, Riemen, Klettband oder dergleichen hat den Vorteil, dass die Orthese beim ersten Anlegen von Fachpersonal speziell an einen Patienten angepasst werden kann und der Patient in der Folge nur noch den Verschluss lösen muss. Die einmal vorgenommenen Einstellungen bleiben somit auch bei mehrmaligem Öffnen und Schließen des Verschlusses erhalten.

Damit auf den Verschluss möglichst geringe Belastungen während des Tragens der Orthese wirken, ist nach einer Weiterbildung vorgesehen, dass die Schlaufenaufnahme ausgehend von der Basis gegenüberliegend zu dem Aufnahmebereich für den Halteabschnitt angeordnet ist, insbesondere an einem entgegengesetzten Ende zu dem Aufnahmebereich für den Halteabschnitt angeordnet ist. Der zwischen der Aussparung des Halteabschnitts und dem Rastelement der Stecklaschen gebildete Hinterschnitt ist damit möglichst weit von der Schlaufenaufnahme und von an der Schlaufenaufnahme wirkenden Kräften entfernt. Einem unerwünschten Aufhebeln der Stecklasche mit dem Rastelement ist somit entgegengewirkt.

Eine nochmalige Verringerung der auf den Verschluss wirkenden Kräfte, insbesondere an der Verbindung zwischen Halteabschnitt und Stecklaschen wirkenden Kräfte, kann nach einer Weiterbildung dadurch sichergestellt werden, dass die Schlaufenaufnahme über eine bewegliche Verbindung mit der Basis des Aufsteckteils verbunden ist. Je nach Ausführung des Verschlusses kann die bewegliche Verbindung auf unterschiedliche Weise erfolgen. Bei einem einteiligen Aufsteckteil kann die bewegliche Verbindung nach einer Weiterbildung zum Beispiel ein flexibler Steg sein und bei einem zweiteiligen oder mehrteiligen Aufsteckteil kann die bewegliche Verbindung zum Beispiel eine Gelenkverbindung sein.

Der flexible Steg kann sich dann beispielsweise dadurch auszeichnen, dass dieser aus einem gegenüber dem restlichen Aufsteckteil dünneren und damit biegsameren Material gefertigt ist.

Die Gelenkverbindung kann auf einfache Weise durch eine der Basis zugeordnete Drehachse gebildet sein, an der die Teile des Aufsteckteils zusammengeführt sind, insbesondere drehbeweglich miteinander verbunden sind. Die Gelenkverbindung kann gemäß vorteilhaften Ausgestaltungen entweder durch eine durch die Schlaufenaufnahme und die Basis geführte Steckachse gebildet sein oder die Schlaufenaufnahme weist einen eine Achse ausbildenden Abschnitt auf, der drehbeweglich in der Basis verklemmt wird, insbesondere in einer Achsführung der Basis verklemmt wird.

Um das Aufsteckteil auf einfache Weise an dem Halteabschnitt festsetzen zu können, ist erfindungsgemäß vorgesehen, dass die Aussparung einer Kante des Rahmens oder des Gerüsts oder der Schiene oder einem anderen Teil, insbesondere formstabilen Teil, der Orthese zugeordnet ist und an der Kante im Bereich der Aussparung eine Aufsteckteilführung ausgebildet ist. Neben einem entsprechend geformten, insbesondere in Richtung Basis breiter werdenden Rastelements dient die Aufsteckteilführung einem einfacheren Zusammenführen des Aufsteckteils und des Halteabschnitts des Verschlusses. Rastelement und Aufsteckteilführung wirken dabei vorteilhafterweise derart zusammen, dass das Aufsteckteil zu der Aussparung des Halteabschnitts zentriert wird.

Die Aufsteckführung wird erfindungsgemäß durch einen sich in Richtung der Aussparung verengenden Aufnahmetrichter, insbesondere durch einen sich auf die Breite der Aussparung verengenden Aufnahmetricher, gebildet.

Das Rastelement gleitet dann beim Schließen des Verschlusses durch den Aufnahmetrichter geführt in Richtung der Aussparung des Halteabschnitts. Die Aussparung und der Aufnahmetrichter sind dabei vorteilhafterweise durch einen Haltesteg des Halteabschnitts voneinander getrennt. Der Haltesteg bildet dann auch den Hinterschnitt, an dem das Rastelement festgesetzt wird, insbesondere verrastet. Um einen optimalen Halt des Rastelements in der Aussparung zu gewährleisten, kann das Rastelement eine maximale Breite aufweisen, die geringfügig kleiner ist als die Breite der Aussparung, insbesondere geringfügig kleiner ist als eine maximale Breite der Aussparung.

Der Aufnahmetrichter der Aufsteckteilführung kann auf einfache Weise durch wulstartige Seitenbereiche des Halteabschnitts gebildet sein, an die sich an den von dem Aufnahmetrichter abgewandten Seiten der Kante jeweils konkave Randabschnitte des Halteabschnitts anschließen. Die wulstartig ausgebildeten Seitenbereiche erstrecken sich dabei in Richtung eines auf den Halteabschnitt aufzuschiebenden Aufsteckteils. Die konkav verlaufende Kante des Halteabschnitts ermöglicht ein Schwenken des Aufsteckteils, wobei das Aufsteckteil durch seine Breite an der Basis und den Radius der konkav verlaufenden Kante in seinem Schwenkbereich begrenzt ist.

Zusätzlich zu dem Rastelement kann an der dem Rastelement gegenüberliegenden Stecklasche ein Führungssteg angeordnet sein, der sich ausgehend von der Basis in Richtung Rastelement erstreckt. Dieser Führungssteg ist an die Aufsteckteilführung, insbesondere den Aufnahmetrichter der Aufsteckteilführung, angepasst, so dass das auf den Halteabschnitt aufgeschobene und verrastete Aufsteckteil mit dem Führungssteg in dem Aufnahmetrichter zwischen den wulstartigen Seitenbereichen der Kante des Halteabschnitts angeordnet ist.

Weiter betrifft die Erfindung auch eine Orthese mit einem Rahmen, einem Gerüst oder einer Schiene, die sich dadurch auszeichnet, dass sie wenigstens einen oben beschriebenen Verschluss aufweist.

Ein Ausführungsbeispiel der Erfindung, aus dem sich weitere erfindungswesentliche Merkmale ergeben können, ist in der Zeichnung dargestellt. Es zeigen:
- Figur 1:: eine Seitenansicht einer erste Ausführung eines Aufsteckteils des erfindungsgemäßen Verschlusses;
- Figur 2:: eine Draufsicht auf die erste Ausführung des Aufsteckteils des erfindungsgemäßen Verschlusses;
- Figur 3:: eine perspektivische Darstellung eines Halteabschnitts des erfindungsgemäßen Verschlusses;
- Figur 4:: eine perspektivische Darstellung des an dem Halteabschnitt gemäß Figur 3 befestigten Aufsteckteils gemäß Figur 1 und Figur 2;
- Figur 5:: ein Querschnitt durch den Halteabschnitt und das Aufsteckteil gemäß Figur 4;
- Figur 6:: eine Explosionsdarstellung einer zweiten Ausführung eines Aufsteckteils des erfindungsgemäßen Verschlusses;
- Figur 7:: eine Seitenansicht des zusammengesetzten Aufsteckteils gemäß Figur 6;
- Figur 8:: eine Explosionsdarstellung einer dritten Ausführung eines Aufsteckteils des erfindungsgemäßen Verschlusses;
- Figur 9:: eine Seitenansicht des zusammengesetzten Aufsteckteils gemäß Figur 8;
- Figur 10:: eine Explosionsdarstellung einer vierten Ausführung eines Aufsteckteils des erfindungsgemäßen Verschlusses;
- Figur 11:: eine Seitenansicht des zusammengesetzten Aufsteckteils gemäß Figur 10;
- Figur 12:: eine perspektivische Darstellung einer fünfte Ausführung eines Aufsteckteils des erfindungsgemäßen Verschlusses; und
- Figur 13:: eine erfindungsgemäße Orthese mit einem erfindungsgemäßen Verschluss.

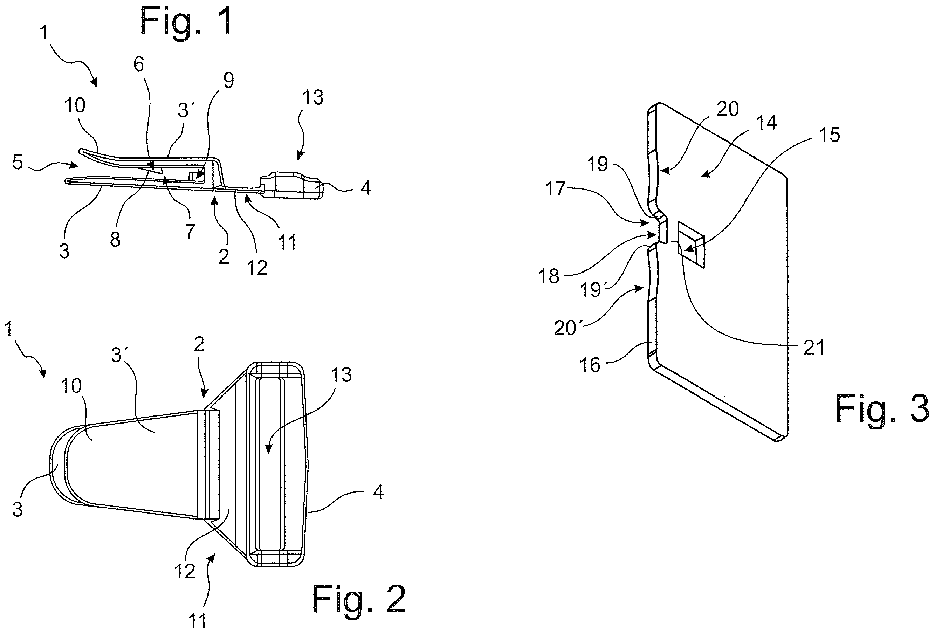

In Figur 1 ist eine erste Ausführung eines Aufsteckteils 1 gezeigt. Dieses Aufsteckteil 1 weist eine Basis 2 auf, die mittig zwischen zwei einander entgegengesetzten Seiten des Aufsteckteils 1 angeordnet ist. Ausgehend von der Basis 2 erstrecken sich zu einer ersten Seite zwei Stecklaschen 3, 3' und zu einer zweiten Seite eine Schlaufenaufnahme 4. Die Stecklaschen 3, 3' sind parallel zueinander ausgerichtet und begrenzen einen Aufnahmebereich 5 des Aufsteckteils 1. An den einander zugewandten Seiten der Stecklaschen 3, 3' weist die Stecklasche 3 ein Rastelement 6 auf. Dieses Rastelement 6 weist einen in Richtung der Basis 2 weisenden Vorsprung 7 auf, wobei zwischen einander zugewandten Flächen der Stecklasche 3, insbesondere einer Innenseite der Stecklasche 3, an der das Rastelement 6 angeordnet ist, und des Rastelements 6 selbst ein Winkel kleiner 90° ausgebildet ist. Mit einer der Stecklasche 3' zugewandten Rückseite 8 weist das Rastelement 6 einen gegenüber den Stecklaschen 3, 3' wesentlich flacheren Winkel auf als mit der der Stecklasche 3 zugewandten Fläche. An der Stecklasche 3' ist weiterhin ein Führungssteg 9 vorgesehen, der sich ausgehend von der Basis 2 in Richtung Rastelement 6 erstreckt. Sowohl der Führungssteg 9 als auch das Rastelement 6 ragen dabei mit ihren gegenüber den Stecklaschen 3, 3' jeweils am Weitesten vorstehenden Bereichen bis an eine zwischen den Stecklaschen 3, 3' gebildete Mittenlinie in den Aufnahmebereich 5 hinein.

An der Stecklasche 3 ist zudem ein Anfasser 10 ausgebildet, mit dem die Stecklasche 3 und damit der Aufnahmebereich 5 aufgebogen werden können, um den Verschluss zu öffnen bzw. zu lösen. Dieser Anfasser 10 ist an einem der Basis 2 entgegengesetzt angeordneten, freien Ende der Stecklasche 3 angeordnet.

Die Schlaufenaufnahme 4 ist über eine bewegliche Verbindung 11, welche als flexibler Steg 12 ausgeführt ist mit der Basis 2 verbunden. Weiter weist die Schlaufenaufnahme 4 eine insbesondere aus der Draufsicht in Figur 2 ersichtliche Ausnehmung 13 auf. Diese Ausnehmung 13 dient der Durchführung einer Schlaufe, eines Riemens, eines Klettbandes oder dergleichen, mit denen dann eine den Verschluss aufweisende Orthese an einem Körperteil festlegbar ist.

In Figur 2 sind, wie auch in den übrigen Figuren, gleiche Teile mit gleichen Bezugszeichen wie in Figur 1 bezeichnet. Auffällig in Figur 2 ist dabei weiter, dass die Stecklaschen 3, 3' zu ihren freien Enden zunehmend schmaler werden und die Schlaufenaufnahme 4 mit der Ausnehmung 13 eine insgesamt wesentlich größere Breite als die Stecklaschen 3, 3' aufweist. Der flexible Steg 12 weist dabei eine trapezförmige Grundfläche auf, die einerseits an die maximale Breite der Basis 2 angepasst ist und andererseits an die Abmessung der Schlaufenaufnahme 4 angepasst ist. Im Bereich des Anfassers 10 am freien Ende der Stecklasche 3 weist diese zudem eine abgerundete Form auf, welche eine ergonomische Bedienung ermöglicht.

Aus Figur 3 geht ein als Gegenstück zu dem Aufsteckteil 1 ausgebildeter Halteabschnitt 14 hervor. Dieser Halteabschnitt 14 weist eine Aussparung 15 auf, die einer Kante 16 des Halteabschnitts 14 zugeordnet ist. Die Kante 16 des Halteabschnitts 14 ist zu einer Aufsteckteilführung 17 geformt. Die Aufsteckteilführung 17 setzt sich aus einem Aufnahmetrichter 18, wulstartigen Seitenbereichen 19, 19' und an die Seitenbereiche 19, 19' anschließenden konkaven Randabschnitten 20, 20' zusammen. Der Aufnahmetrichter 18 der Aufsteckteilführung 17 und die Aussparung 15 sind weiter derart zueinander angeordnet, dass diese fluchtend zueinander ausgerichtet sind, wobei sich der Aufnahmetrichter 18 in Richtung der Aussparung 15 auf die Breite der Aussparung 15 verengt. Aussparung 15 und Aufnahmetrichter 18 sind zudem über einen Haltesteg 21 voneinander getrennt, wobei der Haltesteg 21 bei aufgesetztem Aufsteckteil 1 einen Hinterschnitt für das Rastelement 6 bildet.

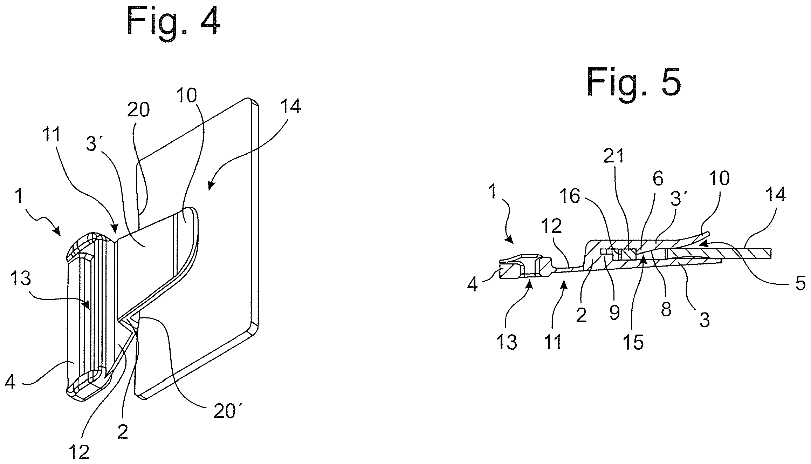

In den folgenden Figuren 4 und 5 sind Aufsteckteil 1 und Halteabschnitt 14 jeweils miteinander verbunden dargestellt, so dass Figur 4 und Figur 5 den geschlossenen Verschluss zeigen. Die Stecklaschen 3, 3' umgreifen dabei den Halteabschnitt 14 zu beiden Seiten des Halteabschnitts 14, der in dem Aufnahmebereich 5 aufgenommen ist.

Aus der geschnittenen Darstellung der Figur 5 geht weiter insbesondere hervor, wie Halteabschnitt 14 und Aufsteckteil 1 ineinandergreifen. Der Halteabschnitt 14 weist eine Dicke auf, die dem Abstand der Stecklaschen 3, 3' zueinander entspricht, insbesondere gleich der Dicke des Aufnahmebereichs 5 ist. Das Rastelement 6 ist in der Aussparung 15 angeordnet und an dem Haltesteg 21 verklemmt. Der Haltestellen 21 ist zwischen Rastelement 6 und Führungssteg 9 angeordnet.

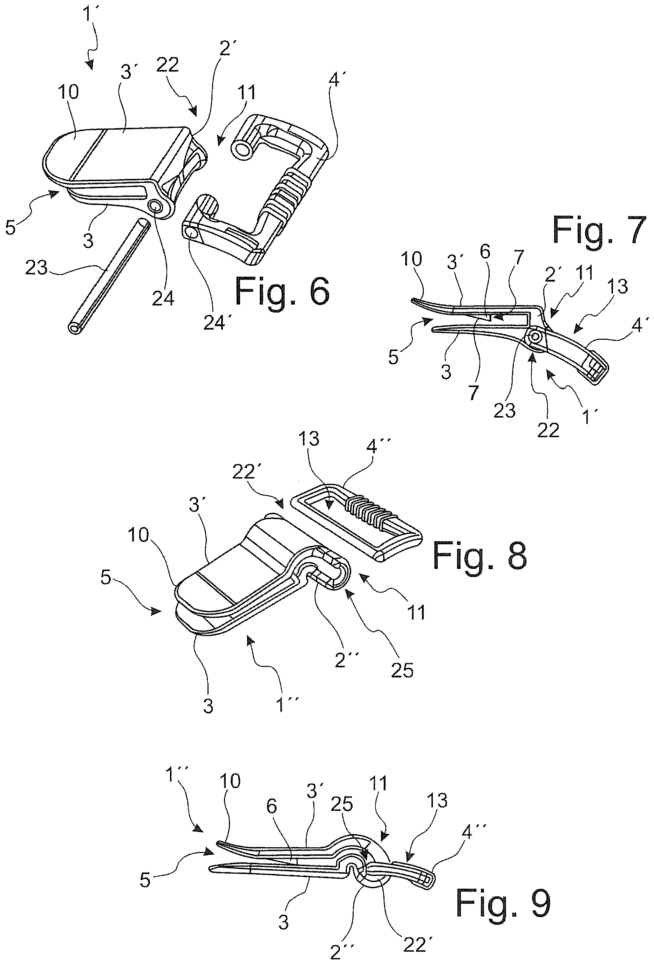

Eine zweite Ausführungsform des Aufsteckteils 1' ist in Figur 6 dargestellt. Diese zweite Ausführungsform unterscheidet sich von dem Aufsteckteil 1 im Wesentlichen dadurch, dass als bewegliche Verbindung 11 eine Gelenkverbindung 22 mit einer Steckachse 23 vorgesehen ist, die in einer Achsdurchführung 24, 24' durch die Basis 2 und wenigstens einem der Basis 2 zugeordneten Teil der Schlaufenaufnahme 4' hindurchgeführt werden kann.

Das zusammengesetzte Aufsteckteil 1' ist in Figur 7 dargestellt. Aus dieser Darstellung wird eine ergonomische Krümmung des Aufsteckteils 1' deutlich, wobei sowohl die Stecklasche 3' als auch die Schlaufenaufnahme 4 ergonomisch gekrümmt sind. Zudem ist die Stecklasche 3 länger ausgeführt als die Stecklasche 3'. Dieser längere, überstehende Stecklaschen-Abschnitt dient als Orientierungshilfe bzw. Einführungshilfe beim Befestigen des Aufsteckteils 1'. Ein Anwender legt diesen verlängerten Stecklaschen-Abschnitt auf den Halteabschnitt 14, 14' und hat in der Höhe das Aufsteckteil 1' direkt positioniert.

In Figur 8 ist eine dritte Ausführung des Aufsteckteils 1" dargestellt. Die dritte Ausführung des Aufsteckteils 1" zeigt wie die zweite Ausführung des Aufsteckteils 1' eine zweiteilige Variante bei der die Basis 2" mit den Stecklaschen 3, 3' einerseits sowie die Schlaufenaufnahme 4" andererseits jeweils einen Teil des Aufsteckteils 1" bilden. Anstatt einer gesonderten Achse oder Steckachse ist hier ein Abschnitt der Schlaufenaufnahme 4" als Achse einer Gelenkverbindung 22' ausgebildet. Dieser Abschnitt der Schlaufenaufnahme 4" wird wie in Figur 9 gezeigt durch den Aufnahmebereich 5 hindurch in einer in die Basis 2" integrierten Achsführung 25 eingesetzt und ist schwenkbeweglich in dieser Achsführung 25 gehaltert. In der Basis 2" ist dazu eine sich an den Aufnahmebereich 5 anschließende, gekrümmte Ausnehmung als Achsführung 25 vorgesehen.

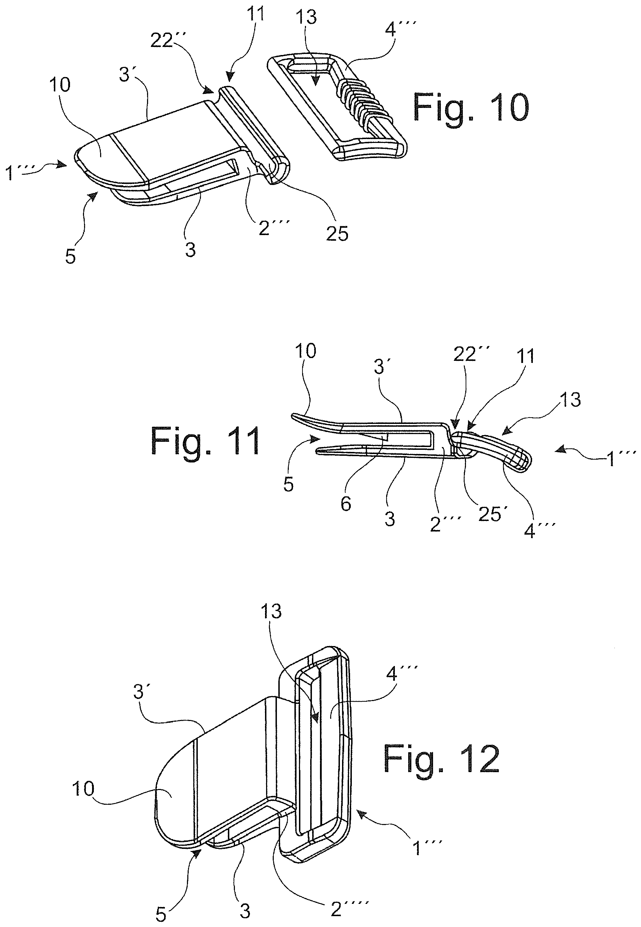

Figur 10 und Figur 11 zeigen schließlich eine vierte Ausführung des Aufsteckteils 1‴. Diese vierte Ausführung verwendet die gleiche Schlaufenaufnahme 4" wie in Figur 8 und Figur 9. Im Unterschied zur vorangegangenen dritten Ausführung ist hier eine Achsführung 25' als Gelenkverbindung 22" der beweglichen Verbindung 11 vorgesehen, die seitlich zu der Basis 2‴ an der von dem Aufnahmebereich 5 abgewandten Seite der Basis 2‴ angeordnet ist. Die Schlaufenaufnahme 4" muss somit bei der vierten Ausführung des Aufsteckteils 1‴ nicht über den Aufnahmebereich 5 beziehungsweise durch den Aufnahmebereich 5 hindurch eingesetzt werden.

Eine fünfte Ausführung des Aufsteckteils 1ʺʺ zeigt Figur 12. Die Ausführung gemäß Figur 12 ist eine besonders einfache Variante ohne bewegliche Verbindung zwischen Basis 2ʺʺ und Schlaufenaufnahme 4‴. Die Schlaufenaufnahme 4‴ ist vielmehr unmittelbar an die Basis 2ʺʺ angesetzt.

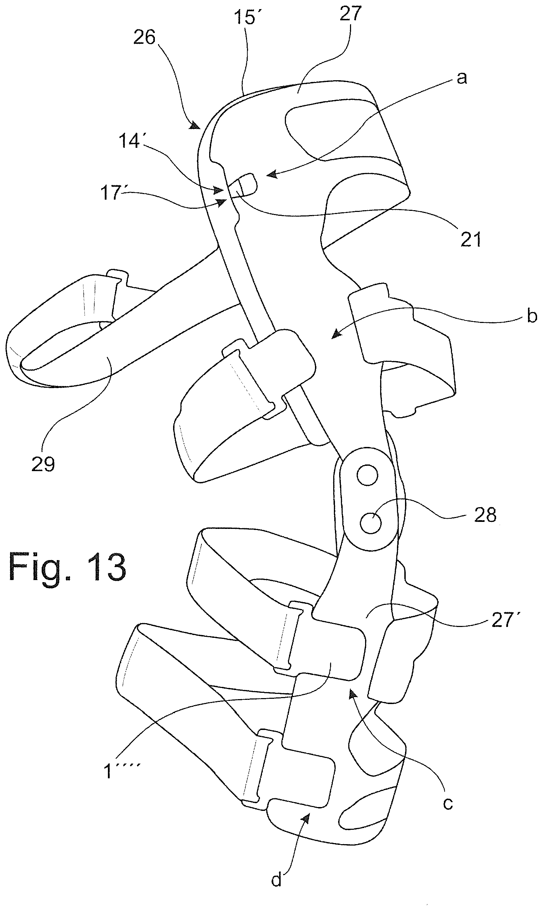

Figur 13 zeigt eine Orthese zur Behandlung eines Körperteils eines Patienten mit insgesamt vier erfindungsgemäßen Verschlüssen a, b c, d. Diese Orthese weist ein Gerüst 26 zur Stabilisierung des Körperteils auf, welches gleichzeitig Halteabschnitte 14' der Verschlüsse a, b, c, d bildet. Das Gerüst 26 setzt sich aus zwei über ein Gelenk 28 miteinander verbundenen Teilen 27, 27' zusammen, wobei jeder Teil 27, 27' des Gerüsts 26 zwei der Verschlüsse a, b, c, d und dementsprechend zwei Halteabschnitte 14' aufweist. Ein oberer Teil 27 des Gerüsts 26 weist die Verschlüsse a, b auf und ein unterer Teil 27' des Gerüsts 26 die Verschlüsse c, d. Jedem Verschluss a, b, c, d ist ein Halteabschnitt 14' und ein Aufsteckteil 1ʺʺ zugeordnet. Die Aufsteckteile 1"" sind über deren Schlaufenaufnahme 4‴ jeweils mit einem Schlaufenband 29 verbunden, welches in seiner Länge an einen Patienten anpassbar ist. Während die Verschlüsse c, d jeweils in geschlossener Position gezeigt sind, sind die Verschlüsse a, b geöffnet bzw. teilweise geöffnet dargestellt. An dem vollständig geöffnet dargestellten Verschluss a ist besonders gut der Aufbau des Halteabschnitts 14' mit seiner Aufsteckteilführung 17' und seiner Aussparung 15' zu erkennen. Diese Aufsteckteilführung 17' unterscheidet sich dadurch, dass diese durch eine Ausbuchtung entlang der Kante 16 des Gerüsts 26 entsprechend der Breite des Aufsteckteils 4' gebildet ist. Die Aussparung 15' ist dabei über einen Haltesteg 21 von der Ausbuchtung getrennt und mittig zu dieser angeordnet.

## Patentansprüche

1. Verschluss (a, b, c, d) für eine Orthese, insbesondere Schnellverschluss für eine Orthese, aufweisend ein Aufsteckteil (1, 1', 1", 1‴, 1ʺʺ) und einen Halteabschnitt (14, 14'), wobei das Aufsteckteil (1, 1', 1", 1‴, 1ʺʺ) mit dem Halteabschnitt (14, 14') lösbar befestigbar ist, wobei das Aufsteckteil (1, 1', 1", 1‴, 1ʺʺ) wenigstens zwei Stecklaschen (3, 3') aufweist, die an einem ersten Ende an einer Basis (2, 2', 2", 2‴, 2ʺʺ) des Aufsteckteils (1, 1', 1", 1‴, 1ʺʺ) miteinander verbunden sind und einen Aufnahmebereich (5) des Aufsteckteils (1, 1', 1", 1‴, 1ʺʺ) zu zwei einander gegenüberliegenden Seiten begrenzen, wobei der Halteabschnitt (14, 14') derart ausgebildet ist, dass dieser bei an diesem befestigten Aufsteckteil (1, 1', 1", 1‴, 1ʺʺ) in dem Aufnahmebereich (5) zwischen den zwei Stecklaschen (3, 3') gehalten ist, wobei der Halteabschnitt (14, 14') Teil eines Rahmens oder eines Gerüsts (26) oder einer Schiene oder eines anderen Teils einer Orthese ist, wobei der Halteabschnitt (14, 14') wenigstens eine Aussparung (15, 15') aufweist, wobei wenigstens eine der Stecklaschen (3, 3') wenigstens ein Rastelement (6) aufweist, und wobei das Rastelement (6) bei auf den Halteabschnitt (14, 14') aufgeschobenem Aufsteckteil (1, 1', 1", 1‴, 1ʺʺ) in die einen Hinterschnitt ausbildende Aussparung (15, 15') eingreift,
**dadurch gekennzeichnet,**
**dass** die Aussparung (15, 15') einer Kante (16) des Rahmens oder des Gerüsts (26) oder der Schiene oder des anderen Teils einer Orthese zugeordnet ist und an der Kante (16) im Bereich der Aussparung (15, 15') eine Aufsteckteilführung (17, 17') ausgebildet ist, und
**dass** die Aufsteckteilführung (17, 17') durch einen sich in Richtung der Aussparung (15) verengenden Aufnahmetrichter (18) gebildet ist.

2. Verschluss (a, b, c, d) nach Anspruch 1, **dadurch gekennzeichnet, dass** das Rastelement (6) wenigstens einen in Richtung der Basis (2, 2', 2", 2‴, 2"") des Aufsteckteils (1, 1', 1", 1‴, 1ʺʺ) weisenden Vorsprung (7) aufweist, wobei zwischen einander zugewandten Flächen der Stecklasche an der das Rastelement (6) angeordnet ist und des Rastelements (6) selbst ein Winkel kleiner 90° ausgebildet ist.

3. Verschluss (a, b, c, d) nach Anspruch 1 oder 2, **dadurch gekennzeichnet, dass** das Rastelement (6) mit seiner der gegenüberliegend angeordneten Stecklasche (3) zugewandten Rückseite (8) in Richtung Basis (2, 2', 2", 2'", 2"") zunehmend breiter wird und einen Keil ausbildet.

4. Verschluss (a, b, c, d) nach einem der Ansprüche 1 bis 3, **dadurch gekennzeichnet, dass** wenigstens eine der Stecklaschen (3, 3') an ihrem, dem ersten Ende entgegengesetzten freien Ende als Anfasser (10) ausgebildet ist.

5. Verschluss (a, b, c, d) nach einem der Ansprüche 1 bis 4, **dadurch gekennzeichnet, dass** wenigstens eine der Stecklaschen (3, 3') länger ist als die andere Stecklasche (3, 3').

6. Verschluss (a, b, c, d) nach einem der Ansprüche 1 bis 5, **dadurch gekennzeichnet, dass** das Aufsteckteil (1, 1', 1", 1‴, 1ʺʺ) wenigstens eine Schlaufenaufnahme (4, 4', 4", 4‴) aufweist.

7. Verschluss (a, b, c, d) nach Anspruch 6, **dadurch gekennzeichnet, dass** die Schlaufenaufnahme (4, 4', 4", 4‴) ausgehend von der Basis (2, 2', 2", 2‴, 2ʺʺ) gegenüberliegend zu dem Aufnahmebereich (5) für den Halteabschnitt (14, 14') angeordnet ist.

8. Verschluss (a, b, c, d) nach einem der Ansprüche 6 oder 7, **dadurch gekennzeichnet, dass** die Schlaufenaufnahme (4, 4', 4", 4‴) über eine bewegliche Verbindung (11) mit der Basis (2, 2', 2", 2‴, 2ʺʺ) des Aufsteckteils (1 , 1', 1", 1‴, 1ʺʺ) verbunden ist.

9. Verschluss (a, b, c, d) nach Anspruch 8, **dadurch gekennzeichnet, dass** die bewegliche Verbindung (11) bei einem einteiligen oder mehrteiligen Aufsteckteil (1, 1"") über einen flexiblen Steg (12) erfolgt und bei einem zweiteiligen Aufsteckteil (1', 1", 1‴) über eine Gelenkverbindung (22, 22', 22") erfolgt.

10. Verschluss (a, b, c, d) nach einem der vorhergehenden Ansprüche, **dadurch gekennzeichnet, dass** der Aufnahmetrichter (18) durch wulstartige Seitenbereiche (19, 19') gebildet ist, an die sich an den von dem Aufnahmetrichter (18) abgewandten Seiten der Kante (16) jeweils konkave Randabschnitte (20, 20') des Halteabschnitts (14) anschließen.

11. Verschluss (a, b, c, d) nach einem der Ansprüche 1 bis 10, **dadurch gekennzeichnet, dass** an der dem Rastelement (6) gegenüberliegenden Stecklasche (3) ein Führungssteg (9) angeordnet ist, der sich ausgehend von der Basis (2, 2', 2", 2‴, 2ʺʺ) in Richtung Rastelement (6) erstreckt.

12. Orthese mit einem Rahmen, einem Gerüst (26) oder einer Schiene,
**dadurch gekennzeichnet,**
**dass** diese wenigstens einen Verschluss (a, b, c, d) nach Anspruch 1 bis 11 aufweist.

## Claims

1. A closure (a, b, c, d) for an orthosis, in particular a quick-release closure for an orthosis, comprising an attachment part (1, 1', 1", 1‴, 1ʺʺ) , and a retaining portion, wherein the attachment part(1, 1', 1", 1‴, 1ʺʺ) can be detachably fastened to the retaining portion (14, 14'), wherein the attachment part (1, 1', 1", 1‴, 1"") has at least two tuck-in flaps (3, 3') which are connected to one another at a first end on a base (2, 2', 2", 2‴, 2ʺʺ) of the attachment part (1, 1', 1", 1‴, 1ʺʺ) and delimit a receptacle region (5) of the attachment part (1, 1', 1", 1‴, 1ʺʺ) with respect to two opposite sides, wherein the retaining portion (14, 14') is designed such that, when the attachment part (1, 1', 1", 1‴, 1ʺʺ) is fastened thereto, it is held in the receptacle region (5) between the two tuck-in flaps (3, 3'), wherein the retaining portion (14, 14') is part of a frame or a framework (26) or a rail or another part of an orthosis, wherein the retaining portion (14, 14') has at least one recess (15, 15'), wherein at least one of the tuck-in flaps (3, 3') has at least one latching element (6), and wherein the latching element (6) engages in the recess (15, 15') forming an indentation when the attachment part (1, 1', 1", 1‴, 1ʺʺ) is slid onto the retaining portion (14, 14') ,
**characterized in that**
the recess (15, 15') is associated with an edge (16) of the frame or of the framework (26) or of the rail or of the other part of an orthosis, and an attachment part guide (17, 17') is formed on the edge (16) in the region of the recess (15, 15'), and
**in that** the attachment part guide (17, 17') is formed by a receiving funnel (18) narrowing in the direction of the recess (15).

2. The closure (a, b, c, d) according to claim 1, **characterized in that** the latching element (6) has at least one projection (7) pointing in the direction of the base (2, 2', 2", 2‴, 2ʺʺ) of the attachment part (1, 1', 1", 1‴, 1ʺʺ), wherein an angle of less than 90° is formed between mutually facing surfaces of the tuck-in flap on which the latching element (6) is arranged and of the latching element (6) itself.

3. The closure (a, b, c, d) according to claim 1 or 2, **characterized in that** the latching element (6) with its rear side (8) facing the oppositely arranged tuck-in flap (3) becomes increasingly wider in the direction of the base (2, 2', 2", 2"', 2ʺʺ) and forms a wedge.

4. The closure (a, b, c, d) according to any one of claims 1 to 3, **characterized in that** at least one of the tuck-in flaps (3, 3') is formed as a grip tab (10) at its free end opposite the first end.

5. The closure (a, b, c, d) according to any one of claims 1 to 4, **characterized in that** at least one of the tuck-in flaps (3, 3') is longer than the other tuck-in flap (3, 3') .

6. The closure (a, b, c, d) according to any one of claims 1 to 5, **characterized in that** the attachment part (1, 1', 1", 1‴, 1ʺʺ) has at least one loop receptacle (4, 4', 4", 4‴, 4ʺʺ).

7. The closure (a, b, c, d) according to claim 6, **characterized in that**, starting from the base (2, 2', 2", 2‴, 2ʺʺ), the loop receptacle (4, 4', 4", 4‴, 4ʺʺ) is arranged opposite the receptacle region (5) for the retaining portion (14, 14').

8. The closure (a, b, c, d) according to any one of claims 6 or 7, **characterized in that** the loop receptacle (4, 4', 4", 4‴, 4ʺʺ) is connected to the base (2, 2', 2", 2‴, 2ʺʺ) of the attachment part (1, 1', 1", 1‴, 1ʺʺ) via a movable connection (11) .

9. The closure (a, b, c, d) according to claim 8, **characterized in that** in the case of a one-piece or multi-piece attachment part (1, 1ʺʺ), the movable connection (11) is made via a flexible web (12) and in the case of a two-piece attachment part (1', 1", 1‴), is made via a hinged connection (22, 22', 22") .

10. The closure (a, b, c, d) according to any one of the preceding claims 11, **characterized in that** the receiving funnel (18) is formed by bead-like side regions (19, 19') which, on the sides of the edge (16) facing away from the receiving funnel (18), are adjoined in each case by concave rim portions(20, 20') of the retaining portion (14).

11. The closure (a, b, c, d) according to any one of claims 1 to 10, **characterized in that** a guide web (9) is arranged on the tuck-in flap (3) opposite the latching element (6), which guide web, starting from the base (2, 2', 2", 2‴, 2ʺʺ), extends in the direction of the latching element (6).

12. An orthosis with a frame, a framework (26) or a rail, **characterized in that**
the orthosis comprises at least one closure (a, b, c, d) according to claims to 11.

## Revendications

1. Elément de fermeture (a, b, c, d) pour une orthèse, en particulier élément de fermeture rapide pour une orthèse, comportant une partie enfichable (1, 1', 1", 1‴, 1ʺʺ) et une section de maintien (14, 14'), sachant que la partie enfichable (1, 1', 1", 1‴, 1ʺʺ) peut être fixée de façon amovible à la section de maintien (14, 14'), sachant que la partie enfichable (1, 1', 1", 1‴, 1ʺʺ) comporte au moins deux languettes d'enfichage (3, 3'), qui sont reliées l'une à l'autre à une première extrémité à une base (2, 2', 2", 2‴, 2ʺʺ) de la partie enfichable (1, 1', 1", 1‴, 1ʺʺ) et délimitent une zone de réception (5) de la partie enfichable (1, 1', 1, 1‴, 1ʺʺ) par rapport aux deux côtés opposés l'un à l'autre , sachant que la section de maintien (14, 14') est constituée de telle manière que celle-ci est maintenue dans la zone de réception (5) entre les deux languettes d'enfichage (3, 3') pour une partie enfichable (1, 1', 1", 1‴, 1ʺʺ) fixée sur celle-ci, sachant que la section de maintien (14, 14') fait partie d'un cadre ou d'une structure (26) ou d'une coulisse ou d'une autre partie d'une orthèse, sachant que la section de maintien (14, 14') comporte au moins un évidement (15, 15'), sachant qu'au moins une des languettes d'enfichage (3, 3') comporte au moins un élément d'encliquetage (6) et sachant que l'élément d'encliquetage (6) vient en prise dans l'évidement (15, 15') constituant une contre-dépouille pour la partie enfichable (1, 1', 1'', 1''', 1ʺʺ) repoussée sur la section de maintien (14, 14'),
**caractérisé en ce que**
l'évidement (15, 15') est attribué à un bord (16) du cadre ou de la structure (26) ou de la coulisse ou de l'autre partie d'une orthèse et un guidage de partie enfichable (17, 17') est constitué sur le bord (16) dans la zone de l'évidement (15, 15'), et
**en ce que** le guidage de partie enfichable (17, 17') est formé par un entonnoir de réception (18) se rétrécissant en direction de l'évidement (15).

2. Elément de fermeture (a, b, c, d) selon la revendication 1, **caractérisé en ce que** l'élément d'encliquetage (6) comporte au moins une saillie (7) tournée en direction de la base (2, 2', 2", 2‴, 2ʺʺ) de la partie enfichable (1, 1', 1", 1‴, 1ʺʺ), sachant qu'un angle inférieur à 90° est constitué entre les surfaces tournées l'une vers l'autre de la languette d'enfichage, sur laquelle est disposé l'élément d'encliquetage (6), et de l'élément d'encliquetage (6) même.

3. Elément de fermeture (a, b, c, d) selon la revendication 1 ou 2, **caractérisé en ce que** l'élément d'encliquetage (6) devient de plus en plus large en direction de la base (2, 2', 2", 2‴, 2ʺʺ) avec son côté arrière (8) tourné vers la languette d'enfichage (3) disposée de façon opposée et constitue un coin.

4. Elément de fermeture (a, b, c, d) selon l'une quelconque des revendications 1 à 3, **caractérisé en ce qu'**au moins une des languettes d'enfichage (3, 3') est constituée sous la forme d'une patte de préhension (10) sur son extrémité libre opposée à la première extrémité.

5. Elément de fermeture (a, b, c, d) selon l'une quelconque des revendications 1 à 4, **caractérisé en ce qu'**au moins une des languettes d'enfichage (3, 3') est plus longue que l'autre languette d'enfichage (3, 3').

6. Elément de fermeture (a, b, c, d) selon l'une quelconque des revendications 1 à 5, **caractérisé en ce que** la partie enfichable (1', 1'', 1''', 1'''') comporte au moins un logement à boucle (4, 4', 4'', 4‴) .

7. Elément de fermeture (a, b, c, d) selon l'une quelconque des revendications précédentes, **caractérisé en ce que** le logement à boucle (4, 4', 4", 4‴) est disposé de façon opposée à la zone de réception (5) pour la section de maintien (14, 14') en partant de la base ((2, 2' , 2", 2‴, 2ʺʺ).

8. Elément de fermeture (a, b, c, d) selon l'une quelconque des revendications 6 ou 7, **caractérisé en ce que** le logement à boucle (4, 4', 4'', 4''') est reliée par une liaison mobile (11) à la base (2, 2', 2", 2‴, 2ʺʺ) de la partie enfichable (1, 1', 1", 1‴, 1ʺʺ).

9. Elément de fermeture (a, b, c, d) selon la revendication 8, **caractérisé en ce que** la liaison mobile (11) a lieu pour une partie enfichable (1, 1ʺʺ) en une partie ou en plusieurs parties par le biais d'une nervure souple (12) et a lieu pour une partie enfichable en deux parties (1', 1", 1‴) par le biais d'une liaison articule (22, 22', 22").

10. Elément de fermeture (a, b, c, d) selon l'une quelconque des revendications précédentes, **caractérisé en ce que** l'entonnoir de réception (18) est formé par des zones latérales en forme de bourrelet (19, 19') auxquelles se raccordent des sections de bordure (20, 20') respectivement concaves de la section de maintien (14) sur les côtés du bord (16) éloignés de l'entonnoir de réception (18).

11. Elément de fermeture (a, b, c, d) selon l'une quelconque des revendications 1 à 10, **caractérisé en ce que** sur la languette d'enfichage (3) opposée à l'élément d'encliquetage (6) est disposée une nervure de guidage (9), qui s'étend en direction de l'élément d'encliquetage (6) en partant de la base (2, 2', 2", 2‴, 2ʺʺ).

12. Orthèse avec un cadre, une structure (26) ou une coulisse,
**caractérisé en ce que** celle-ci comporte au moins une élément de fermeture (a, b, c, d) selon l'une quelconque des revendications 1 à 11.
